# EUROPÄISCHE PATENTANMELDUNG

(11) **EP 4 154 842 A1**
(43) Veröffentlichungstag der Anmeldung: **29.03.2023**
(21) Anmeldenummer: 21198394.5
(22) Anmeldetag: 22.09.2021
(51) Int. Cl.: A61C 7/00, B29C 64/171, B33Y 70/00, B33Y 80/00, C08K 5/101, C08K 5/09, C08L 33/08, C08L 33/10, A61K 6/60, A61K 6/62, A61K 6/887, A61C 5/00, A61C 7/08

(54) **3D-DRUCKHARZ MIT SEPARATIONSEFFEKT**

(71) Anmelder: PRO3DURE MEDICAL GMBH, 44227 Dortmund (DE)
(72) Erfinder: KLARE, Martin, 44227 Dortmund (DE); KUNZ, Heiner Julian, 44139 Dortmund (DE); GISCHER, Frank, 58708 Menden (DE)
(74) Vertreter: Schulz Junghans Patentanwälte PartGmbB

(57) **Zusammenfassung**

Die vorliegende Erfindung betrifft ein 3D-Druckharz umfassend ein Monomer oder Oligomer, welches mindestens eine Acrylat- oder Methacrylat-Untereinheit aufweist, und einen Initiator. Das 3D-Druckharz ist dadurch charakterisiert, dass es eine gesättigte Fettsäure oder einen Carbonsäureester umfasst, wodurch ein Separationseffekt gegenüber anderen Kunststoffen erzielt wird. Die Erfindung bezieht sich ferner auf ein Polymer, das die oben beschriebenen monomere und Oligomere in polymerisierter Form sowie eine gesättigte Fettsäure oder einen Carbonsäureester aufweist. Werden lediglich 60-80 % der Doppelbindungen des Monomers oder Oligomers umgesetzt, kann ein Grünling erhalten werden. Durch die Erfindung wird auch ein Kit-of-Parts-System bereitgestellt, welches das oben beschriebene 3D-Druckharz zur Herstellung eines Polymers mit Separationseffekt sowie eine Pulver- und eine Flüssigkomponente zur Herstellung eines Kunststoffes umfasst. Schließlich bezieht sich die Erfindung auch noch auf Verfahren zur Herstellung eines Formteils mit Separationseffekt sowie auf ein Verfahren zur Herstellung eines Formteils, wobei das Formteil komplementär zu einem anderen Formteil ist. Bei letzterem Verfahren wird ein Formteil aus dem oben beschriebenen 3D-Druckharz gefertigt und das andere Formteil aus einem Kunststoff.

## Beschreibung

Die vorliegende Erfindung bezieht sich auf ein 3D-Druckharz mit Separationseffekt sowie daraus hergestellte Formteile und deren Herstellungsverfahren.

### Hintergrund der Erfindung

Ziel der vorliegenden Erfindung ist es, ein 3D-Druckharz mit einem inhärenten Separationseffekt gegenüber weiteren Kunststoffsystemen zur Verfügung zu stellen. 3D gedruckte Objekte aus dem erfindungsgemäßen Material können im Medizintechnikbereich und insbesondere im Dentalbereich in Kombination mit traditionellen Herstellungsmethoden vorteilhaft angewandt werden. Als Beispiel hierfür sei die Herstellung von kieferorthopädischen Apparaturen mittels der so genannten "Salz und Pfeffer" Technik genannt. Dabei wird auf einem Dentalmodell ein Pulver-Flüssigsystem schrittweise aufgetragen indem kleine Mengen Pulver (vorzugsweise Polymethylmethacrylatpolymerpulver) mit monomeren Methylmethacrylat beträufelt werden, bis sich eine pastöse Konsistenz einstellt. Das Pulver/Flüssigkeitssystem ist mit einem Initiatorsystem versetzt, so dass in seltenen Fällen eine direkte Autopolymerisation auf dem Dentalmodell stattfindet oder in den meisten Fällen das Modell mit dem pastösen, gestreuten Objekt in einen Drucktopf gegeben wird und unter Druck und Hitze aushärtet. Traditionell kommen dazu Dentalmodelle aus Gips zum Einsatz, die mit einer Isolatorlösung besprüht werden, bevor auf diesen der Streukunststoff appliziert wird. Nach der Aushärtung können dann die Objekte aus dem Pulver/Flüssigsystem einfach vom Gipsmodell getrennt und ausgearbeitet (z.B. beschliffen und poliert) werden.

Heutzutage findet jedoch ein Wechsel zu "digitalen Workflows" auf der Basis von biometrischen Daten statt. Das heißt, dass auf der Basis von Scandaten ein 3D-gedrucktes Dentalmodell die Arbeitsgrundlage für den Zahntechniker bildet. Solche Modelle bestehen nicht mehr aus Gips, sondern werden aus Kunstharzen in einem Schichtbauprozess (z.B. mittels eines Bildprojektionssystems wie dem ASIGA MAX UV der Fa. Asiga, Sydney) hergestellt. Daraus ergeben sich gleich mehrere Problemstellungen. Zum einen kann die Monomerkomponente des Pulver-Flüssigsystems das Polymernetzwerk des Dentalmodellkunststoffes anlösen, in das Netzwerk eindringen und ein "interpenetrating network" bilden. Nach der Aushärtung besteht dann ein Verbund mit dem Dentalmodell und das Objekt kann nicht mehr ohne Schaden vom Modell gelöst werden. Zum anderen bestehen solche 3D-gedruckten Modelle aus Schichten und besitzen deshalb keine glatten, sondern inhomogene Oberflächen. Daraus können zusätzlich mechanische Retentionen entstehen, die durch das "Aufreißen des Isolatorfilmes" an z.B. Unterschnitten oder flachen Anstiegen des Modells verstärkt werden. Darüber hinaus ist die Benetzung solcher Polymeroberflächen nur vollständig, wenn ein auf Oberflächeneigenschaften des Kunststoffes optimiertes Isolatorsystem eingesetzt wird. Dementsprechend kommen heutzutage u.a. Gele mit Separationseigenschaften (z.B. glycerinbasierte Gele) zum Einsatz. Das händische Auftragen eines solchen Gels ist zum einen zeitaufwändig und zum anderen immer inhomogen, insbesondere an Unterschnitten und in Interdentalräumen. Darüber hinaus ist es extrem mühsam eine vollständige Beschichtung der Arbeitsgrundlage sicherzustellen. Infolgedessen kann somit nicht immer vermieden werden, dass es einen Verbund zwischen Dentalmodell und dem Streukunststoff nach der Aushärtung gibt. Schlimmstenfalls brechen beim Ablösen dann Teile des Gerätes ab und der Prozess muss komplett wiederholt werden. Als weiterer wichtiger Nachteil dieser Vorgehensweise ist anzuführen, dass durch unterschiedliche Schichtdicken und Inhomogenitäten beim Auftragen des Separatorgels eine Oberfläche für den Streukunststoff entsteht, die nicht mehr präzise die anatomischen Gegebenheiten repräsentiert. Daraus können beispielsweise Passungsproblem resultieren, die eine Nachbearbeitung oder sogar eine Neuanfertigung des kieferorthopädischen Gerätes erfordern.

Um all den o.g. Nachteilen zu begegnen, ist es deshalb wünschenswert, einen Modellkunststoff zur Verfügung zu stellen, der einen inhärenten Separationseffekt besitzt, welcher zu einer homogenen, gegenüber Streukunststoffen isolierenden Oberfläche der gedruckten Objekte führt. Stand der Technik ist dazu das 3D-Druckharz "e-Sepfree" der Fa. Envisiontec, Gladbeck. Dem Material sind als Füller Wachspartikel zugesetzt, die zu einer verminderten Haftung von Streukunststoffen an aus diesem Harz gebauten Objekten führt. Diese spezielle Eigenschaft der Wachspartikel gegenüber flüssigen Monomeren/Polymeren führt allerdings auch dazu, dass in einem flüssigen 3D-Druckharz eine Separierung der Partikel stattfindet und die Wachspartikel im Harz zu Boden sinken. Daraus können fehlerhafte Baujobs resultieren bzw. Bauteile mit inhomogenen Oberflächeneigenschaften entstehen. Ferner muss das Harz regelmäßig durchmischt werden, um die o.g. Fehlerquellen zu vermeiden. Darüber hinaus sind mit Wachspartikeln gefüllte 3D-Druckharze opak. Je nach gewählter 3D-Drucktechnologie (z.B. Stereolithographie mit Bestrahlungsquelle von oben und Absinken des Bauteiles in das Harz) kann so während des Bauvorganges keine Bauprozesskontrolle vorgenommen werden. Entsprechend können so fehlerhafte Bauvorgänge nicht rechtzeitig abgebrochen bzw. während des Bauens modifiziert (durch z.B. Löschen eines Bauteils) und so "gerettet" werden.

Als weitere Nachteile sind zusätzlich anzuführen, dass durch die Wachspartikel zum einen die Viskosität des Bauharzes ansteigt und die mechanischen Kennwerte erniedrigt werden.

Die vorliegende Erfindung stellt eine Lösung der o.g. Probleme und Einschränkungen zur Verfügung. Es wurde überraschend gefunden, dass sich aus 3D-Druckharzen, die >5m% einer gesättigten Fettsäure bzw. ihrer Ester mit einer Kettenlänge <20 C enthalten, generativ gefertigte Objekte herstellen lassen, die einen im Vergleich zum Stande der Technik signifikant erhöhten, inhärenten Separationseffekt aufweisen. Die beanspruchten Harzformulierungen sind homogen. Insbesondere weisen die erfindungsgemäßen Harze keine Separations- bzw. Segregationseffekte auf. Darüber hinaus können auf der Basis der Erfindung Harzsysteme zur Verfügung gestellt werden, die transparent sind und die sich nach der Polymerisation weiß opak verfärben. Dadurch können die generierten Objekte während des Baujobs optimal im Harzvat beobachtet und so eine optimale Prozesskontrolle gewährleistet werden. Im Vergleich zu mit Wachspartikeln gefüllten Systemen ist die Viskosität der erfindungsgemäßen Harze signifikant niedriger. Dies ist für 3D-Druckprozesse aus vielerlei Gründen vorteilhaft. Auf der einen Seite läuft das Harz (in Abhängigkeit von der Technologie) nach dem Bau jeder Schicht einfacher nach und benetzt das Bauteil schneller. So lassen sich höhere Baugeschwindigkeiten erzielen. Zum anderen lassen sich die additiv gefertigten Bauteile leichter und effizienter reinigen, wenn die Viskosität niedriger ist.

### Zusammenfassung der Erfindung

Ein erster Aspekt der Erfindung bezieht sich auf ein 3D-Druckharz umfassend
- ein Monomer oder Oligomer, welches mindestens eine Acrylat- oder Methacrylat-Untereinheit aufweist,
- einen Initiator,
dadurch charakterisiert, dass das 3D-Druckharz eine gesättigte Fettsäure oder einen Carbonsäureester umfasst.

Ein zweiter Aspekt der Erfindung bezieht sich auf ein Polymer umfassend polymerisierte Monomere und/oder Oligomere gemäß dem ersten Aspekt, und eine gesättigte Fettsäure oder einen Carbonsäureester.

Ein dritter Aspekt der Erfindung bezieht sich auf einen Grünling umfassend polymerisierte Monomere und/oder Oligomere gemäß dem ersten Aspekt, wobei 60 % bis 80 % der Doppelbindungen der Acrylat- und/oder Methacrylatuntereinheiten umgesetzt worden sind, und eine gesättigte Fettsäure oder einen Carbonsäureester.

Ein vierter Aspekt der Erfindung bezieht sich auf Kit-of-parts-System umfassend
- ein 3D-Druckharz gemäß dem ersten Aspekt zur Herstellung eines Polymers mit einem Separationseffekt, und
- eine Pulverkomponente und eine Flüssigkomponente zur Herstellung eines Kunststoffes, wobei die Pulverkomponente ein Polymerpulver umfasst, und die Flüssigkomponente ein Monomer umfasst.

Ein fünfter Aspekt der Erfindung bezieht sich auf ein Verfahren zur Herstellung eines Formteils mit einem Separationseffekt, wobei das 3D-Druckharz gemäß dem ersten Aspekt verwendet und ausgehärtet wird.

Ein sechster Aspekt der Erfindung bezieht sich auf ein Verfahren zur Herstellung eines Formteils, wobei
- in einem ersten Schritt das Verfahren gemäß dem fünften Aspekt durchgeführt wird, um ein erstes Formteil zu erhalten, und
- in einem zweiten Schritt das erste Formteil verwendet wird, um ein zweites Formteil zu erhalten, das mindestens abschnittweise komplementär zum ersten Formteil ist, wobei das zweite Formteil aus einem Kunststoff gefertigt wird,
   oder
- in einem ersten Schritt ein erstes Formteil aus einem Kunststoff gefertigt wird, und
- in einem zweiten Schritt das erste Formteil verwendet wird, um ein zweites Formteil zu erhalten, das mindestens abschnittweise komplementär zum ersten Formteil ist, wobei das zweite Formteil unter Verwendung des 3D-Druckharzes gemäß dem ersten Aspekt hergestellt wird.

### Begriffe und Definitionen

Der Begriff *Alkyl* richtet sich auf eine gesättigte lineare oder verzweigte Kohlenwasserstoffkette. Ein C₁₋₄-Alkyl bezeichnet eine Kohlenwasserstoffkette, welche 1, 2, 3 oder 4 Kohlenstoffatome umfasst. Beispiele für C₁₋₄-Alkyle sind Methyl, Ethyl, Propyl, Isopropyl, n-Butyl, 2-Methylpropyl, *tert*-Butyl.

Der Begriff *Alkenyl* richtet sich auf eine lineare oder verzweigte Kohlenwasserstoffkette, die neben Kohlenstoff-Kohlenstoff-Einfachbindungen mindestens eine Kohlenstoff-Kohlenstoff-Doppelbindung aufweist.

Der Begriff *Cycloalkyl* oder *cycloaliphatisch* bezieht sich auf eine gesättigte mono- oder polyzyklische Kohlenwasserstoffverbindung. Monozyklische Kohlenwasserstoffverbindungen bilden eine Ringstrruktur aus, z.B. Cyclohexyl (-C₆H₁₁). Polyzyklische Kohlenwasserstoffverbindungen umfassen Kohlenwasserstoffverbindungen, die mehrere Ringe ausbilden, z.B. Isobornyl oder Tricyclodecyl. Ein C₃₋₁₈-Cycloalkyl bezieht sich auf eine mono- oder polyzyklische Kohlenwasserstoffverbindung, die 3 bis 18 Kohlenstoffatome umfasst. Cycloalkyle können mit einem oder mehreren C₁₋₄-Alkylen substituiert sein, z.B. Tricyclodecanedimethanol.

Der Begriff *heterocyclisch* bezieht sich auf eine mono- oder polyzyklische Kohlenwasserstoffverbindung, bei der mindestens ein Kohlenstoffatom durch ein Heteroatom, insbesondere N, O oder S, ausgetauscht ist. Insbesondere sind Kohlenstoffatome nur über Einfachbindungen (gesättigte heterocyclische Verbindung) oder über Einfachbindungen und Doppelbindungen miteinander verbunden.

Der Begriff *Initiator* richtet sich auf Moleküle, die ein Radikal bilden und somit eine Polymerisationsreaktion starten. Die Radikalbildung wird durch sog. *Aktivatoren* katalysiert.

Der Begriff *Aktivator* bezieht sich auf chemische Verbindungen, eine Temperaturerhöhung, Licht oder energiereiche Strahlung, die einen Initiator zur Radikalbildung anregen. Beispielsweise können Peroxidverbindungen thermisch oder photochemisch zur Radikalbildung angeregt werden.

Der Begriff *interpenetrierendes Netzwerk* richtet sich auf ein Polymernetzwerk umfassend zwei oder mehrere Netzwerke, die zumindest teilweise auf molekularer Ebene vernetzt, aber nicht kovalent miteinander verbunden sind. Die Netzwerke können nicht getrennt werden, es sei denn, chemische Bindungen werden gebrochen.

Der Begriff *Dimerfettsäure, dimerisierte Fettsäure oder Dimersäure* bezieht sich auf ein Gemisch, das durch Oligomerisierung, insbesondere Dimerisierung, von Fettsäuren (Verkochung) hergestellt wurde. Für die Oligomerisierung ist mindestens eine Fettsäure mit konjugierten Doppelbindungen sowie weitere ungesättigte Fettsäuren notwendig. Insbesondere werden ungesättigte C₁₂₋₂₂-Fettsäuren eingesetzt. Die Reaktion erfolgt mittels Diels-Alder-Addition, wodurch sich in der Regel ein teilweise ungesättigter C₆-Ring bildet. Je nach Zahl und Lage der Doppelbindungen der zur Herstellung der Dimerfettsäuren eingesetzten Fettsäuren entstehen Gemische aus überwiegend dimeren Produkten. Werden C₁₂₋₂₂-Fettsäuren eingesetzt, weisen die Dimere dann beispielsweise 24 bis 44 Kohlenstoffatome auf. Insbesondere werden zur Herstellung Fettsäuren mit 18 Kohlenstoffatomen eingesetzt, sodass das dimere Produkt 36 Kohlenstoffatome aufweist. Neben dem Dimer können auch Trimere sowie Monomere der Fettsäuren im Gemisch vorliegen. Handelsübliche Dimerfettsäuren enthalten im Allgemeinen mindestens 80 Gew.-% dimere Moleküle, bis zu 19 Gew.-% trimere Moleküle und maximal 1 Gew.-% monomerer Moleküle und sonstiger Nebenprodukte. Insbesondere werden Dimerfettsäuren eingesetzt, die zu mindestens 90 Gew.-%, insbesondere zu mindestens 95 Gew.-%, weiter insbesondere zu mindestens 98 Gew.-% aus dimeren Fettsäuremolekülen bestehen. Geeignete Dimerfettsäuren mit 36 Kohlenstoffatomen sind beispielsweise in dem unter "Empol 1062" bei der Firma Cognis erhältlichen Gemisch enthalten.

Der Begriff *Tinuvin P* bezieht sich auf CAS 2440-22-4.

Der Begriff *Tinopal OB* bezieht sich auf CAS 7128-64-5.

Der Begriff *TPO* bezieht sich auf CAS-Nr.75980-60-8.

Der Begriff *TPOL* bezieht sich auf CAS No. 84434-11-7.

Der Begriff *Irgacure* 819 bezieht sich auf CAS No. 162881-26-7.

### Beschreibung der Erfindung

Ein erster Aspekt der Erfindung bezieht sich auf ein 3D-Druckharz umfassend
- ein Monomer oder Oligomer, welches mindestens eine Acrylat- oder Methacrylat-Untereinheit aufweist,
- einen Initiator,
dadurch charakterisiert, dass das 3D-Druckharz eine gesättigte Fettsäure oder einen Carbonsäureester umfasst.

Das 3D-Druckharz der vorliegenden Erfindung kann zum 3D-Drucken von Objekten verwendet werden, wobei der Initiator aktiviert wird und eine Polymerisationsreaktion abläuft. Die im 3D-Druckharz enthaltenen gesättigten Fettsäuren oder Carbonsäureester bilden einen Film um die Polymerstrukturen, wodurch ein Separationseffekt gegenüber anderen Kunststoffen erzielt wird. Wie oben beschrieben, kann dieser Effekt beispielsweise im Dentalbereich bei der Herstellung von kieferorthopädischen Passstücken hilfreich sein. So kann eine mit Hilfe des 3D-Druckharzes hergestellte Nachbildung des Kiefers inklusive des Gebisses als Positiv-Form für die Herstellung von Zahnschienen genutzt werden. Die Zahnschiene kann durch bekannte Pulver-Flüssigsysteme wie beispielsweise einer Polymerisation von Polymethylmethacrylatpolymer (Pulver) und Methylmethacrylat (Flüssigkomponente) direkt auf der Positiv-Form hergestellt werden und aufgrund des Separationseffekts leicht von der Form gelöst werden.

Das 3D-Druckharz kann eine oder mehrere Arten von Monomeren und/oder Oligomeren aufweisen. Es ist auch eine Kombination mehrerer gesättigter Fettsäuren und/oder Carbonsäureestern möglich.

In einer Ausführungsform ist die Fettsäure eine Säure gemäß Formel (1),

R¹⁰-COOH (1),

wobei R¹⁰ ein C₁₋₉-Alkyl, insbesondere ein C₃₋₁₉-Alkyl, weiter insbesondere ein C₁₁₋₁₇-Alkyl, ist.

In einer Ausführungsform ist der Carbonsäureester ausgewählt aus einem Ester gemäß Formel (2), einem Ester gemäß Formel (3) oder einem Ester, der aus einer Dimerfettsäure und zwei Alkoholen, insbesondere C₋₁₋₁₂-Alkyl-OH, hergestellt worden ist,

R¹¹-C(=O)-O-R¹² (Monocarbonsäureester) (2),

R¹³-O-C(=O)-R¹⁴-C(=O)-O-R¹⁵ (Dicarbonsäureester) (3),

wobei
R¹¹ ein C₁₋₂₃-Alkyl oder ein C₂₋₂₃-Alkenyl, insbesondere ein C₃₋₂₃-Alkyl oder ein C₃₋₂₃-Alkenyl ist, wobei das Alkyl oder das Alkenyl unsubstituiert ist oder durch ein oder mehrere, insbesondere ein, -OH substituiert ist,
R¹², R¹³ und R¹⁵ ein C₁₋₁₂-Alkyl ist,
R¹⁴ ein C₁₋₁₂-Alkyl oder ein C₂₋₁₂-Alkenyl, insbesondere ein C₂₋₁₂-Alkyl oder ein C₂₋₁₂-Alkenyl ist.

Der Carbonsäureester kann einen gesättigten oder ungesättigten Säureanteil enthalten. In der Regel ist der Säureanteil unsubstituiert. Gering substituierte Ester wie beispielsweise ein Rizinolsäureester sind jedoch auch geeignet.

In einer Ausführungsform ist R¹¹ unsubstituiert.

In einer Ausführungsform ist die Dimerfettsäure eine Säure gemäß Formel (5), 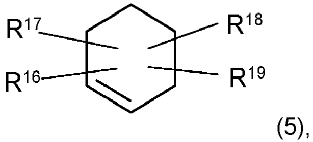 wobei
R¹⁶ und R¹⁷ unabhängig voneinander ein C₁₋₂₃-Alkyl oder ein C₂₋₂₃-Alkenyl, insbesondere ein C₃₋₂₃-Alkyl oder ein C₃₋₂₃-Alkenyl sind, wobei das Alkyl oder das Alkenyl unsubstituiert ist oder durch ein oder mehrere, insbesondere ein, -OH substituiert ist,
R¹⁸ und R¹⁹ unabhängig voneinander C₁₋₂₃-Alkyl-COOH oder C₂₋₂₃-Alkenyl-COOH, insbesondere C₃₋₂₃-Alkyl-COOH oder C₃₋₂₃-Alkenyl-COOH sind, wobei das Alkyl oder das Alkenyl unsubstituiert ist oder durch ein oder mehrere, insbesondere ein, -OH substituiert ist.

In einer Ausführungsform sind R¹⁶, R¹⁷, R¹⁸ und R¹⁹ unsubstitutiert.

In einer Ausführungsform ist die Summe aller C-Atome des Carbonsäureesters ≤ 20, insbesondere 4-20, weiter insbesondere 12-18.

In einer Ausführungsform beträgt der Gewichtsanteil der Fettsäure oder des Carbonsäureesters bezogen auf die Gesamtmasse des 3D-Druckharzes ≥ 5 %.

In einer Ausführungsform ist der Säureanteil des Carbonsäureesters ausgewählt aus:
n-Butansäure (Buttersäure), 2-Methylpropansäure (Isobuttersäure), Pentansäure (Valeriansäure), i-Pentansäure, wie z.B. 2,2-Dimethylpropansäure (Pivalinsäure, Neopentansäure) und 3-Methylbutansäure (iso-Pentansäure, iso-Valeri- ansäure), Hexansäure (Capronsäure), Heptansäure, Octansäure (Caprylsäure), i-Octansäure wie z.B. insbesondere 2- Propylheptyl-2-ethylhexansäure, aber auch 2-Propylheptyl-3-ethylhexansäure 2-Propylheptyl-4-ethylhexan- säure, 2-Propylheptyl-5-ethylhexansäure sowie technische Gemische von verzweigten Octansäuren, wie sie beispielsweise unter dem Handelsnamen Cekanoic^{®} C8 von der Fa. Exxon vertrieben werden. Nonansäure (Pelargonsäure, Nonylsäure), Decansäure (Caprinsäure), i-Decansäuren, wie z.B. Trimethylheptansäure (Neodecansäure, Iso- decansäure) sowie technische Gemische verzweigter Decansäuren, wie sich beispielsweise unter dem Handelsnamen Cekanoic^{®} C10 von der Fa. Exxon vertrieben werden, Undecansäure, Undecensäure, Dodecansäure (Laurinsäure), Tridecansäure, Tetradecansäure (Myristinsäure), Pentadecansäure, Hexadecansäure (Palmitinsäure), Heptadecansäu- re (Margarinsäure), Octadecansäure (Stearinsäure), Nonadecansäure, Eicosansäure, Docosansäure, Tetracosansäure, Hexacosanäure, Dimerfettsäuren (C36, wie beispielsweise unter dem Handelsnamen "Empol 1062" von der Fa. Cognis erhältlich) Talkfettsäuren, Kokosfettsäuren, Palmfettsäuren, Rizinolsäure, Ölsäure, Linolsäure, Linolensäure, Isostea- rinsäure, Isooctansäure, Isononansäure, Isodecansäure, 2-Ethylhexansäure, 2-Propylheptansäure, 2-Butyloctansäure, 2-Butyldecansäure, 2-Hexyloctansäure, 2-Hexyldecansäure, 2-Hexyldodecansäure, 2-Octyldecansäure, Fumarsäure, Maleinsäure, Adipinsäure, Pimelinsäure, Korksäure, Azelainsäure, Sebazinsäure. Geeignet sind auch Ester mit Cekanoic^{®}C8 (Isooctansäure), Cekanoic^{®}C9 (Isononansäure: 3,5,5-Trime- thylhexansäure und 2,5,5-Trimethylhexansäure) und Cekanoic^{®}C10 (Isodecansäure) von der Fa. Exxon Mobile, die Carbonsäure-Isomerengemische darstellen.

In einer Ausführungsform ist der Alkoholanteil des Carbonsäureesters ausgewählt aus Methanol, Ethanol, Propanol, Isopropanol, Butanol, Isobutanol, Pentanol, Hexanol, Isohexanol, Octanol, Decanol und Dodecanol.

In einer Ausführungsform umfasst das Monomer oder Oligomer neben der mindestens einen Acrylat- oder Methacrylat-Untereinheit eine oder mehrere Untereinheiten ausgewählt aus einer aliphatischen, einer cycloaliphatischen, einer heterocyclischen, einer aromatischen und einer Urethan-Untereinheit.

In einer Ausführungsform ist das Monomer aus einer Verbindung der Formel (4) ausgewählt, 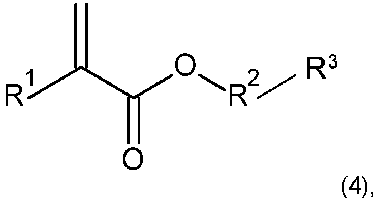 wobei
R¹ gleich -H oder -CH₃ ist,
R² ausgewählt ist aus
   ∘ C₁- bis C₁₀-Alkyl,
   ∘ -[(CH₂)ₘ-O-]ₙ-(CH₂)ᵣ-,
   ∘ -[(C₁₋₁₂-Alkyl)ₛ-Y]ₓ-(CH₂)ₜ-
   ∘ 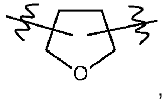
   ∘ 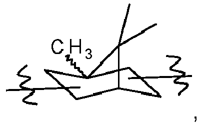
   ∘ 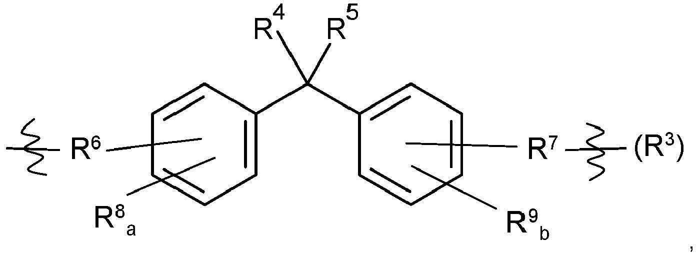
   wobei
   R⁴ und R⁵ unabhängig voneinander ausgewählt sind aus H, -C₁₋₄-Alkyl, -CF₃, phenyl, oder
   R⁴ und R⁵ eine Ringstruktur bilden, umfassend eine Kohlenwasserstoffkette umfassend 4 bis 8 Kohlenstoffatome,
   R⁶ ausgewählt ist aus -[(CH₂)ₚ-O-]_{q}-,
   R⁷ ausgewählt ist aus -O-[(CH₂)ᵥ-O-]_{w}-,
   R⁸ₐ und R⁹_{b} unabhängig voneinander ausgewählt sind aus -C₁₋₄-Alkyl, phenyl,
   Y ausgewählt ist aus -O-C(=O)-NR²⁰- und -NR²⁰-C(=O)-O-,
   R²⁰ ein C₁₋₄-Alkyl oder H ist,
   m, p, v zwischen 1 und 4, insbesondere 1 oder 2, ist,
   n, q, w zwischen 1 und 4 ist,
   r, t, x zwischen 1 und 4 ist, insbesondere 1 oder 2,
   s zwischen 1 und 10 ist,
   a 0 oder 1 ist,
   b 0 oder 1 ist, und
R³ gleich -H, Acrylat oder Methacrylat ist.

In einer Ausführungsform ist das Monomer ausgewählt aus:
- Dimethacrylate des (n)-alkoxylierten Bisphenol A wie Bisphenol-A-ethoxylat(2)dimethacrylat, Bisphenol-A-ethoxylat(4)dimethacrylat, Bisphenol-A-propoxylat(2)dimethacrylat, Bisphenol-A-propoxylat(4)dimethacrylat sowie Dimethacrylate des (n)-alkoxylierten Bisphenol F wie Bisphenol-F-ethoxylat(2)dimethacrylat und Bisphenol-F-ethoxylat(4)dimethacrylat, Bisphenol-F-propoxylat(2)dimethacrylat, Bisphenol-F-propoxylat(4)dimethacrylat und Mischungen dieser. Vorzugsweise verwendet man monomere oder oligomere Dimethacrylate auf Basis von Bisphenol A, insbesondere das Bisphenol-A-ethoxylat(2)dimethacrylat und das Bisphenol-A-ethoxylat(4)dimethacrylat. Besonders bevorzugt sind dabei Mischungen dieser beiden Dimethacrylate auf Bisphenol-A-Basis, mit einem Anteil an Bisphenol-A-ethoxylat(4)dimethacrylat > Bisphenol-A-ethoxylat(2)dimethacrylat.
- Urethan(meth)acrylate mit einer Funktionalität < 4 sind dem Fachmann bekannt und können in bekannter Weise hergestellt werden, indem man beispielsweise ein hydroxylterminiertes Polyurethan mit Methacrylsäure zum entsprechendem Urethanmethacrylat umsetzt, oder indem man ein isocyanatterminiertes Präpolymer mit Hydroxymethacrylaten umsetzt. Entsprechende Verfahren sind z.B. aus EP 0579503 bekannt. Urethan(meth)acrylate sind auch im Handel erhältlich und werden beispielsweise unter der Bezeichnung PC-Cure® von der Firma Piccadilly Chemicals, unter der Produktbezeichnung CN 1963 von der Firma Sartomer, unter der Bezeichnung Photomer von der Firma Cognis, unter der Bezeichnung Ebecryl von der Firma UCB und unter der Bezeichnung Genomer® von der Firma Rahn vertrieben. Bevorzugt werden als Urethan(meth)acrylate solche eingesetzt, die n < 4 funktionalisiert sind, Viskositäten <15 Pa s besitzen, ein Molekülgewicht < 2000 haben, und aus aliphatischen Edukten hergestellt worden sind. Insbesondere findet das aus HEMA und TMDI erhaltene Isomerengemisch 7,7,9- (bzw. 7,9,9-)Trimethyl-4, 13-dioxo-3, 14-dioxa-5,12-diazahexadecan-1, 16-diol-dimethacrylat Anwendung.
- 1,3-Butandioldimethacrylat, 1,6-Hexandioldimethacrylat, 1,3-Butylenglykoldimethacrylat, Diethylenglykoldimethacrylat, Ethylenglykoldimethacrylat, Neopentyldimethacrylat, Polyethylenglykoldimethacrylat, Triethylenglykoldimethacrylat und Tetraethylenglykoldimethacrylat und bevorzugt 1,4-Butandioldimethacrylat. Solche Produkte sind im Handel erhältlich, beispielsweise von der Firma Sartomer.
- ein Umsetzungsprodukt eines asymmetrisch trimerisierten, aliphatischen Isocyanates mit einem hydroxylgruppenhaltigen Methacrylat, beispielsweise dem 2-Hydroxyethylmethacrylat eingesetzt werden. Solche Produkte sind im Handel erhältlich, beispielsweise von der Firma Rahn. Die asymmetrischen Varianten zeichnen sich durch eine geringere Viskosität im Verhältnis zu den symmetrischen Varianten aus.

In einer Ausführungsform ist das Oligomer aus den oben beschriebenen Monomeren gebildet.

In einer Ausführungsform umfasst das Oligomer XX bis XX Monomere.

Bei Verwendung des 3D-Druckharzes in einem 3D-Drucker erfolgt die Aushärtung/Polymerisation durch UV-Licht. Durch Bestrahlung mit Licht einer geeigneten Wellenlänge wird der Initiator gespalten und es bildet sich ein Radikal aus, wodurch die Polymerisation der Monomere und/oder Oligomere gestartet wird.

In einer Ausführungsform ist der Initiator ein Photoinitiator.

In einer Ausführungsform ist der Initiator ein Photoinitiator ausgewählt aus
∘ einer Verbindung der Benzoine und Benzoinether, insbesondere Benzoin, Benzoinmethylether, Benzoinethylether und Benzoinisopropylether, Benzoinphenylether und Benzoinacetat,
∘ einer Verbindung der Acetophenone, insbesodere Acetophenon, 2,2-Dimethoxyacetophenon, und 1,1-Dichloracetophenon,
∘ einer Verbindung der Benzile und Benzilketale, insbesondere Benzil, Benzildimethylketal und Benzildiethylketal,
∘ einer Verbindung der Anthrachinone, insbesondere 2-Methylanthrachinon, 2-Ethylanthrachinon, 2-tert.-Butylanthrachinon, 1-Chloranthrachinon und 2-Amylanthrachinon,
∘ einer Verbindung der Triphenylphosphine und Benzoylphosphinoxide, insbesondere Triphenylphosphine, 2,4,6-Trimethylbenzoyldiphenylphosphinoxid (Luzirin TPO) und Bis(2,4,6-trimethylbenzoylphenyl)-phosphinoxid,
∘ einer Verbindung der Benzophenone, insbesondere Benzophenon und 4, 4'-Bis-(N, N'-dimethylamino)-benzophenon,
∘ einer Verbindung der Thioxanthone und Xanthone, insbesondere Thioxanton und Xanthon,
∘ einem Acridinderivat, Phenazinderivat, Quinoxalinderivat
∘ 1-Phenyl-1,2-propandion-2-0-benzoyloxim,
∘ einer Verbindung der 1-Aminophenylketone oder 1-Hydroxyphenylketone, insbesondere 1-Hydroxycyclohexylphenylketon, Phenyl-(1-hydroxyisopropyl)-keton und 4-Isopropylphenyl-(1-hydroxyisopropyl)-keton.

Bei 3D-Druckverfahren können Zusätze verwendet werden, die das entweder das hergestellte Objekt modifizieren, z.B. durch Farbmittel, oder den Druckprozess erleichtern. Beispielsweise kann durch UV-Blocker die Eindringtiefe von UV-Strahlung zum Start der Polymerisationsreaktion reguliert werden oder die Stabilität über anaerobe oder aerobe Stabilisatoren beeinflusst werden.

In einer Ausführungsform umfasst das 3D-Druckharz einen oder mehrere Zusätze ausgewählt aus einem Inhibitor, einem Stabilisator, einem Füllstoff, einem Farbmittel, einem UV-Blocker.

Ein zweiter Aspekt der Erfindung bezieht sich auf ein Polymer umfassend polymerisierte Monomere und/oder Oligomere gemäß dem ersten Aspekt, und eine gesättigte Fettsäure oder einen Carbonsäureester.

Das oben beschriebene 3D-Druckharz beinhaltet Monomere und/oder Oligomere, die in einer radikalischen Polymerisationsreaktion ein Polymer ausbilden können.

In einer Ausführungsform ist das Polymer unter Verwendung des 3D-Druckharzes gemäß dem ersten Aspekt der Erfindung hergestellt.

In einer Ausführungsform sind die Monomere und/oder Oligomere vollständig polymerisiert.

Bei der Fertigung von Dentalmodellen kann es hilfreich sein, zunächst einen Grünling herzustellen und diesen dann später vollständig auszuhärten.

Ein dritter Aspekt der Erfindung bezieht sich auf einen Grünling umfassend polymerisierte Monomere und/oder Oligomere gemäß dem ersten Aspekt, wobei 60 % bis 80 % der Doppelbindungen der Acrylat- und/oder Methacrylatuntereinheiten umgesetzt worden sind, und eine gesättigte Fettsäure oder einen Carbonsäureester.

In einer Ausführungsform ist der Gründling unter Verwendung des 3D-Druckharzes gemäß dem ersten Aspekt der Erfindung hergestellt.

Ein vierter Aspekt der Erfindung bezieht sich auf Kit-of-parts-System umfassend
- ein 3D-Druckharz gemäß dem ersten Aspekt der Erfindung zur Herstellung eines Polymers mit einem Separationseffekt, und
- eine Pulverkomponente und eine Flüssigkomponente zur Herstellung eines Kunststoffes, wobei
   ∘ die Pulverkomponente ein Polymerpulver, insbesondere ein Polymerpulver umfassend mindestens eine Acrylat- oder Methacrylat-Untereinheit, umfasst, und
   ∘ die Flüssigkomponente ein Monomer, insbesondere ein Monomer umfassend mindestens eine Acrylat- oder Methacrylat-Untereinheit, umfasst.

In einer Ausführungsform umfasst die Pulverkomponente ein Polymethylmethacrylatpolymer und die Flüssigkomponente Methylmethacrylat.

Das erfindungsgemäße 3D-Druckharz wird insbesondere verwendet, wenn zwei komplementäre Kunststoff-Objekte wie z.B. ein Dentalmodell und eine passgenaue Zahnschiene, hergestellt werden sollen. Damit z.B. die Zahnschiene vom Dentalmodell gelöst werden kann, wird das Dentalmodell unter Verwendung des oben beschriebenen 3D-Druckharzes hergestellt und die Zahnschiene aus einem bekannten Kunststoffsystem, wie beispielsweise einem Pulver-Flüssigsystem (Streukunststoff). Für derartige Fertigungen kann das Kit-of-parts-System verwendet werden. Das Pulver-Flüssigsystem umfasst in der Regel weitere Komponenten wie einen Initiator zum Start der Polymerisationsreaktion und ggf. weitere Zusätze (s. oben). Geeignete Initiatoren sind einer Fachperson bekannt. Aufgrund des Separationseffektes ist es nicht nötig, Isolatorlösungen oder isolierende Gele, die in bekannten Verfahren eine Trennung der komplementären Objekte ermögliche, einzusetzen.

In einer Ausführungsform umfasst das Kit-of-parts-System keine Isolatoren.

Ein fünfter Aspekt der Erfindung bezieht sich auf ein Verfahren zur Herstellung eines Formteils mit einem Separationseffekt, wobei das 3D-Druckharz gemäß dem ersten Aspekt verwendet und ausgehärtet wird.

In einer Ausführungsform wird bei dem Verfahren gemäß dem fünften Aspekt ein 3D-Drucker verwendet.

Ein sechster Aspekt der Erfindung bezieht sich auf ein Verfahren zur Herstellung eines Formteils, wobei
- in einem ersten Schritt das Verfahren gemäß dem fünften Aspekt durchgeführt wird, um ein erstes Formteil zu erhalten, und
- in einem zweiten Schritt das erste Formteil verwendet wird, um ein zweites Formteil zu erhalten, das mindestens abschnittweise komplementär zum ersten Formteil ist, wobei das zweite Formteil aus einem Kunststoff gefertigt wird, insbesondere unter Verwendung einer Pulverkomponente und einer Flüssigkomponente, wobei
   ∘ die Pulverkomponente ein Polymerpulver, insbesondere ein Polymerpulver umfassend mindestens eine Acrylat- oder Methacrylat-Untereinheit, umfasst, und
   ∘ die Flüssigkomponente ein Monomer, insbesondere ein Monomer umfassend mindestens eine Acrylat- oder Methacrylat-Untereinheit, umfasst,
   oder
- in einem ersten Schritt ein erstes Formteil aus einem Kunststoff gefertigt wird, insbesondere unter Verwendung einer Pulverkomponente und einer Flüssigkomponente, wobei
   ∘ die Pulverkomponente ein Polymerpulver, insbesondere ein Polymerpulver umfassend mindestens eine Acrylat- oder Methacrylat-Untereinheit, umfasst, und
   ∘ die Flüssigkomponente ein Monomer, insbesondere ein Monomer umfassend mindestens eine Acrylat- oder Methacrylat-Untereinheit, umfasst, und
- in einem zweiten Schritt das erste Formteil verwendet wird, um ein zweites Formteil zu erhalten, das mindestens abschnittweise komplementär zum ersten Formteil ist, wobei das zweite Formteil unter Verwendung des 3D-Druckharzes gemäß dem ersten Aspekt hergestellt wird.

In einer Ausführungsform wird
- in einem ersten Schritt das Verfahren gemäß dem fünften Aspekt durchgeführt, um ein erstes Formteil zu erhalten, und
- in einem zweiten Schritt das erste Formteil verwendet, um ein zweites Formteil zu erhalten, das mindestens abschnittweise komplementär zum ersten Formteil ist, wobei das zweite Formteil aus einem Kunststoff gefertigt wird, insbesondere unter Verwendung einer Pulverkomponente und einer Flüssigkomponente, wobei
   ∘ die Pulverkomponente ein Polymerpulver, insbesondere ein Polymerpulver umfassend mindestens eine Acrylat- oder Methacrylat-Untereinheit, umfasst, und
   ∘ die Flüssigkomponente ein Monomer, insbesondere ein Monomer umfassend mindestens eine Acrylat- oder Methacrylat-Untereinheit, umfasst.

Das Pulver-Flüssigsystem umfasst in der Regel weitere Komponenten wie einen Initiator zum Start der Polymerisationsreaktion und ggf. weitere Zusätze (s. oben). Geeignete Initiatoren sind einer Fachperson bekannt. Aufgrund des Separationseffektes ist es nicht nötig, Isolatorlösungen oder isolierende Gele, die in bekannten Verfahren eine Trennung der komplementären Objekte ermögliche, einzusetzen.

In einer Ausführungsform umfasst das Verfahren nicht die Verwendung von Isolatoren.

In einer Ausführungsform umfasst die Pulverkomponente ein Polymethylmethacrylatpolymer und die Flüssigkomponente Methylmethacrylat.

### Beschreibung der Figuren

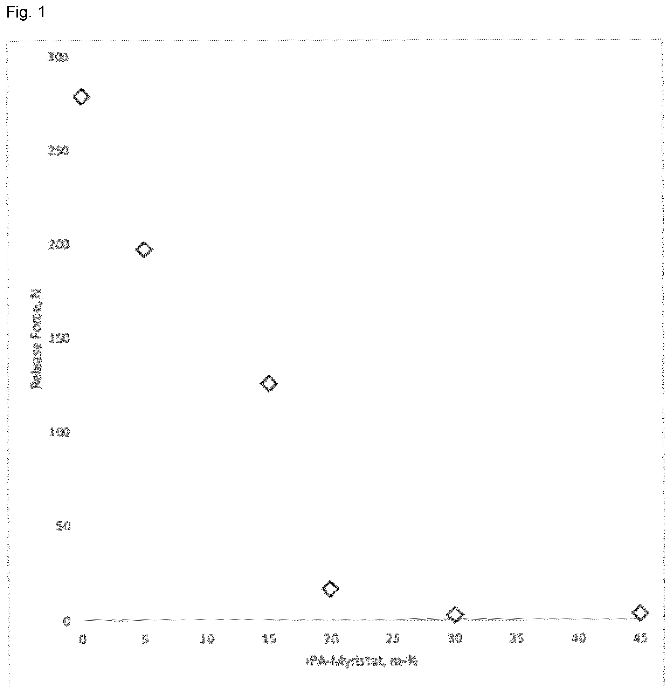

- Fig. 1: zeigt die Ablösbarkeit (Release Forces), N in Abhängigkeit vom Masseanteil IPA-Myristat. Die Probekörper wurden gemäß Tabelle 1 hergestellt.

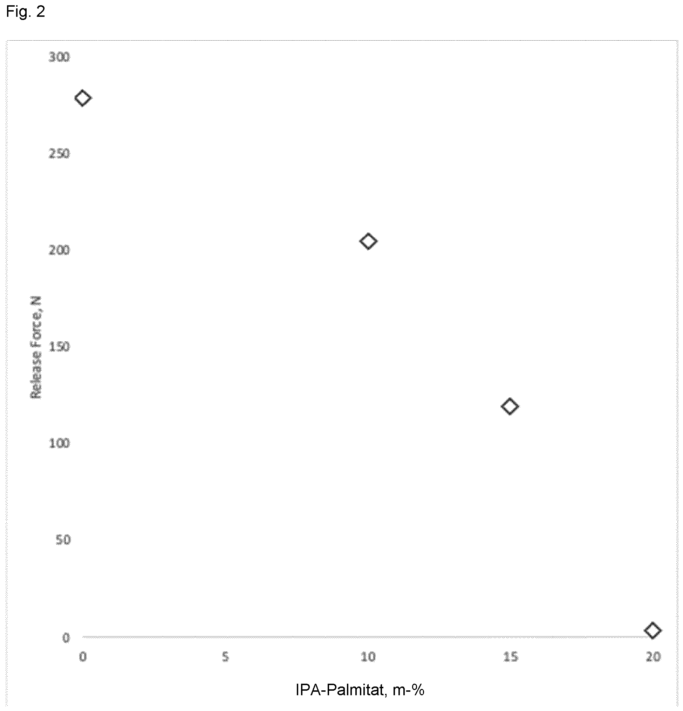

- Fig. 2: zeigt die Ablösbarkeit (Release Forces), N in Abhängigkeit vom Masseanteil IPA-Palmitat. Die Probekörper wurden gemäß Tabelle 2 hergestellt.

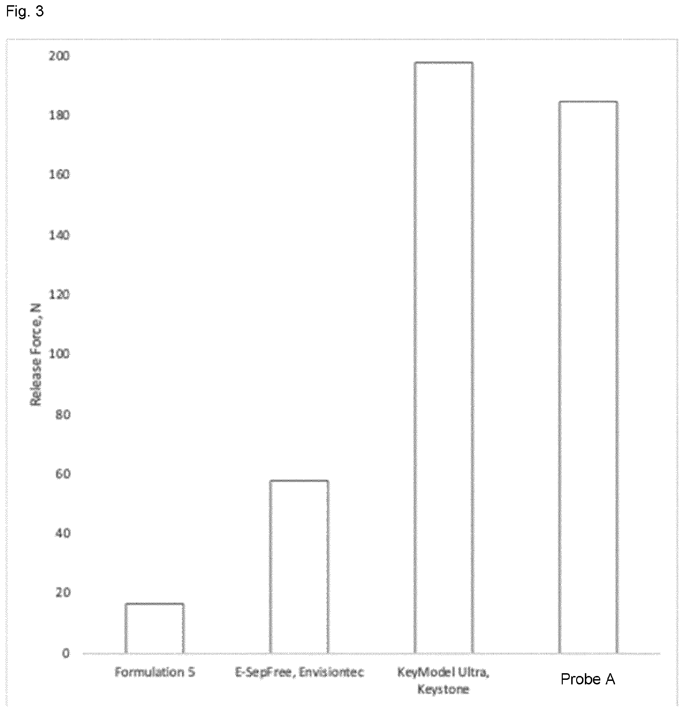

- Fig. 3: zeigt die Ablösbarkeit (Release Forces), N (ISO 22112) der erfindungsgemäßen Formulierung 5 im Vergleich zu E-SepFree, Envisiontec sowie "Standard" Modellmaterialien.

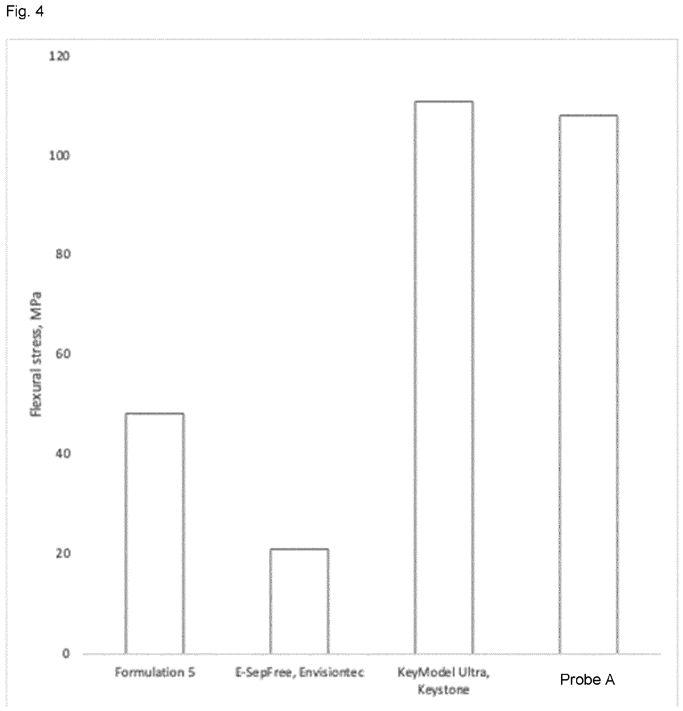

- Fig. 4: zeigt die Biegespannung (Flexural stress) (ISO 178) der erfindungsgemäßen Formulierung 5 im Vergleich zu E-SepFree, Envisiontec sowie "Standard" Modellmaterialien.

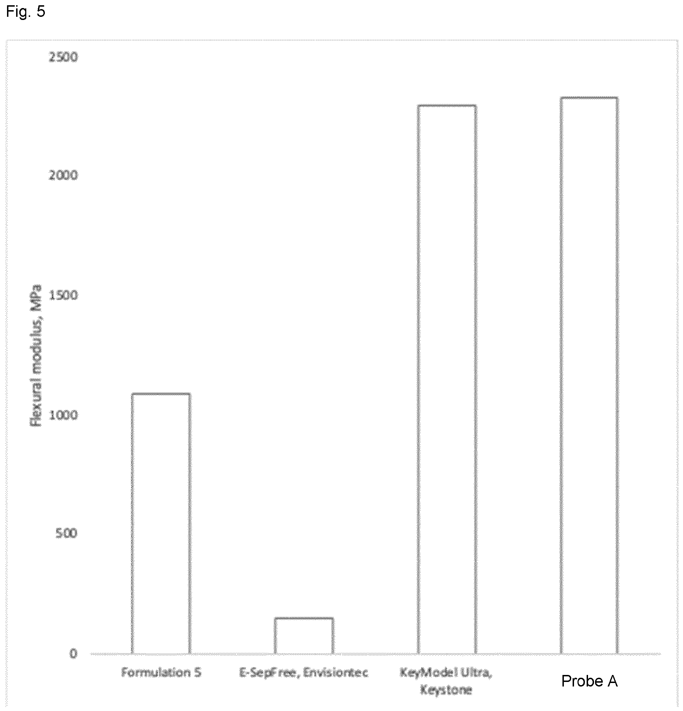

- Fig. 5: zeigt die Biegespannung (Flexural modulus) (ISO 178) der erfindungsgemäßen Formulierung 5 im Vergleich zu E-SepFree, Envisiontec sowie "Standard" Modellmaterialien.

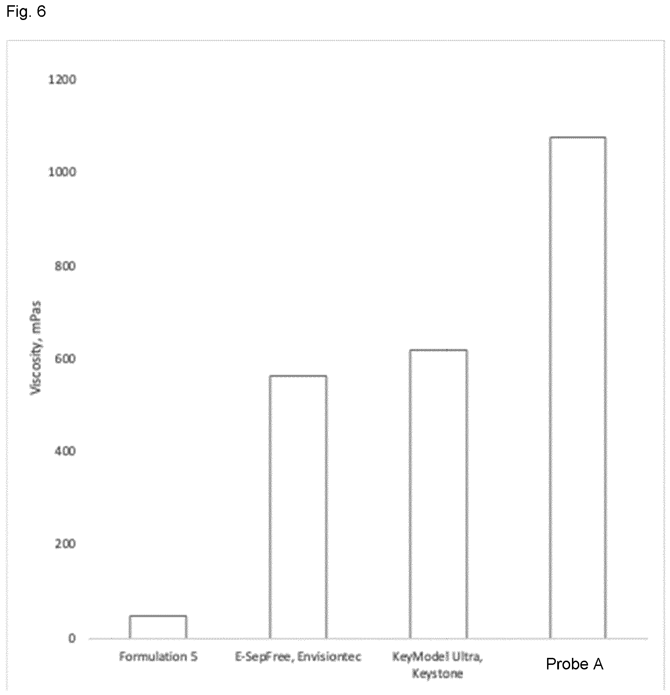

- Fig. 6: zeigt die Viskosität, mPas (DIN 53019-1) der erfindungsgemäßen Formulierung 5 im Vergleich zu E-SepFree, Envisiontec sowie "Standard" Modellmaterialien.

### Beispiele

### Herstellung der Probe A

Die Probe A wurde durch Mischen eines Methacrylatharzes mit Isopropyl-Myrisatat (30 wt%) hergestellt. Das Methacrylatharz bestand aus Tetraethylenglycoldimethacrylat (60 wt%) und einem difunktionellen aliphatischen Methacrylat (MIRAMER PU2421NT, 40 wt%). Bezogen auf das Gewicht der Methacrylate wurden dem Harz TPO (CAS-Nr.75980-60-8) (1.4 wt%), Irgacure 819 (CAS No. 162881-26-7) (0,6 wt%), Tinuvin P (CAS 2440-22-4) (0.1 wt%) und Tinopal OB CO (CAS 7128-64-5) (0.01 wt%) zugegeben. Die Komponenten wurden mit einem Ultraturrax vermischt (30 min, 16000 min^-1).

Das lichthärtende Harz wurde mittels einer Apparatur zur generativen Fertigung (DLP-SL-3D-Drucker: ASIGA MAXUV) verarbeitet.

### Quantifizierung der Haftung

Zum Vergleich der Haftung eines Modellkunststoffes an unterschiedlichen Harzen wurde die maximale Zugkraft bestimmt. Hierzu wurde ein zweiteiliger Probenkörper mittels generativer Fertigung (ASIGA MAXUV) hergestellt. Die zwei Teile (Teil 1: 10×10×5 mm, Teil 2: 10×20×5 mm) wurden orthogonal zueinander verklebt, wobei die Oberseite des Teil 1 (10×10 mm) mit der kurzen Seite des Teil 2 (10×5 mm) L-förmig verbunden wurde. Die Probenkörper wurden bei einem Spaltabstand von 2 mm mittels eines Pulver-Flüssig-Systems (Streukunststoff auf Basis eines Polymethylmethacrylatpolymerpulvers und monomeren Methylmethacrylats) zur Herstellung kieferorthopädischer Vorrichtungen miteinander verbunden (PL-1, pro3dure medical GmbH). Die Messung der Abziehkräfte erfolgte in einer Vorrichtung gemäß ISO 22112. Die Vorlast betrug 0.5 N, die Traversengeschwindigkeit 5 mm/min. Als Ergebnis wurde die Maximalkraft festgehalten.

### Mechanikprüfung

Die Mechanikprüfung erfolgte nach ISO178. Es wurden je 5 Probenkörper (2×2×25 mm) getestet. Die Traversengeschwindigkeit betrug 10 mm/min. Die Berechnung der Ergebnisse erfolgte nach ISO178.

**Tabelle 1: Formulierungen mit Myristinsäure-Isopropylester**

| No. | IUPAC name | Formulation 1 | Formulation 2 | Formulation 3 | Formulation 4 | Formulation 5 | Formulation 6 | Formulation 7 |
|---|---|---|---|---|---|---|---|---|
| 1 | 2-[2-[2-[2-(2-methylprop-2-enoyloxy)ethoxy)ethoxy)ethoxy)ethyl 2-methylprop-2-enoate | 60 | 57 | 54 | 51 | 48 | 42 | 33 |
| 2 | 2[[3,5,5-trimethyl-6[2-(2-methylprop-2-enoyloxy)ethoxycarbonylamino]hexyl)carbamoyloxy]ethyl 2-methylprop-2-enoate | 38 | 36,1 | 34,2 | 32,3 | 30,4 | 26,6 | 20,9 |
| 3 | [phenyl-(2,4,6-trimethylbenzoyl)phosphoryl]-(2,4,6-trimethylphenyl)methanone | 1 | 0,95 | 0,9 | 0,85 | 0,8 | 0,7 | 0,55 |
| 4 | diphenylphosphoryl-(2,4,6-trimethylphenyl)methanone | 1 | 0,95 | 0,9 | 0,85 | 0,8 | 0,7 | 0,55 |
| 5 | propan-2-yl tetradecanoate | 0 | 5 | 10 | 15 | 20 | 30 | 45 |
| | | 100 | 100 | 100 | 100 | 100 | 100 | 100 |

**Tabelle 2: Formulierungen mit Palmitinsäure-Isopropylester**

| No. | IUPAC name | formulation 8, % | formulation 9, % | formulation 10, % | Formulation 11, % |
|---|---|---|---|---|---|
| 1 | 2-[2-[2-[2-(2-methylprop-2-enoyloxy)ethoxy]ethoxy]ethoxy]ethyl 2-methylprop-2-enoate | 60 | 57 | 54 | 48 |
| 2 | 2-[[3,5,5-trimethyl-6-[2-(2-methylprop-2-enoyloxy)ethoxycarbonylamino]hexyl]caramoyloxy]ethyl 2-methylprop-2-enoate | 38 | 36,1 | 34,2 | 30,4 |
| 3 | [phenyl-(2,4,6-trimethylbenzoyl)phosphoryl]-(2,4,6-trimethylphenyl)methanone | 1 | 0,95 | 0,9 | 0,8 |
| 4 | diphenylphosphoryl-(2,4,6-trimethylphenyl)methanone | 1 | 0,95 | 0,9 | 0,8 |
| 5 | propan-2-yl hexadecanoate | 0 | 5 | 10 | 20 |
| | | 100 | 100 | 100 | 100 |

## Patentansprüche

1. Ein 3D-Druckharz umfassend
- ein Monomer oder Oligomer, welches mindestens eine Acrylat- oder Methacrylat-Untereinheit aufweist,
- einen Initiator,
dadurch charakterisiert, dass
das 3D-Druckharz eine gesättigte Fettsäure oder einen Carbonsäureester umfasst.

2. Das 3D-Druckharz nach Anspruch 1, wobei die Fettsäure eine Säure gemäß Formel (1) ist,
R¹⁰-COOH (1),
wobei R¹⁰ ein C₁₋₁₉-Alkyl, insbesondere ein C₃₋₁₉-Alkyl, weiter insbesondere ein C₁₁₋₁₇-Alkyl, ist.

3. Das 3D-Druckharz nach einem der vorhergehenden Ansprüche, wobei der Carbonsäureester ausgewählt ist aus einem Ester gemäß Formel (2), einem Ester gemäß Formel (3) oder einem Ester, der aus einer Dimerfettsäure und zwei Alkoholen, insbesondere C-₁₋₁₂-Alkyl-OH, hergestellt worden ist,
R¹¹-C(=O)-O-R¹² (2),
R¹³-O-C(=O)-R¹⁴-C(=O)-O-R¹⁵ (3),
wobei
R¹¹ ein C₁₋₂₃-Alkyl oder ein C₂₋₂₃-Alkenyl, insbesondere ein C₃₋₂₃-Alkyl oder ein C₃₋₂₃-Alkenyl ist, wobei das Alkyl oder das Alkenyl unsubstituiert ist oder durch ein oder mehrere, insbesondere ein, -OH substituiert ist,
R¹², R¹³ und R¹⁵ ein C₁₋₁₂-Alkyl ist,
R¹⁴ ein C₁₋₁₂-Alkyl oder ein C₂₋₁₂-Alkenyl ist.

4. Das 3D-Druckharz nach einem der vorhergehenden Ansprüche, wobei die Summe aller C-Atome des Carbonsäureesters ≤ 20, insbesondere 4-20, weiter insbesondere 12-18 ist.

5. Das 3D-Druckharz nach einem der vorhergehenden Ansprüche, wobei der Gewichtsanteil der Fettsäure oder des Carbonsäureesters bezogen auf die Gesamtmasse des 3D-Druckharzes ≥ 5 % beträgt.

6. Das 3D-Druckharz nach einem der vorhergehenden Ansprüche, wobei das Monomer oder Oligomer neben der mindestens einen Acrylat- oder Methacrylat-Untereinheit eine oder mehrere Untereinheiten ausgewählt aus einer aliphatischen, einer cycloaliphatischen, einer heterocyclischen, einer aromatischen und einer Urethan-Untereinheit umfasst.

7. Das 3D-Druckharz nach einem der vorhergehenden Ansprüche, wobei das Monomer aus einer Verbindung der Formel (4) ausgewählt ist, 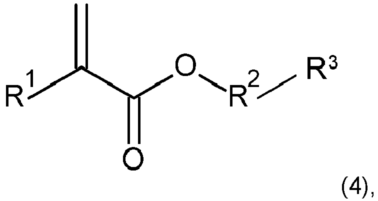 wobei
R¹ gleich -H oder -CH₃ ist,
R² ausgewählt ist aus
∘ C₁- bis C₁₀-Alkyl,
∘ -[(CH₂)ₘ-0-]ₙ-(CH₂)ᵣ-,
∘ -[(C₁₋₁₂-Alkyl)ₛ-Y]ₓ-(CH₂)ₜ-
∘
∘
∘ 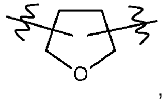 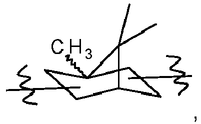 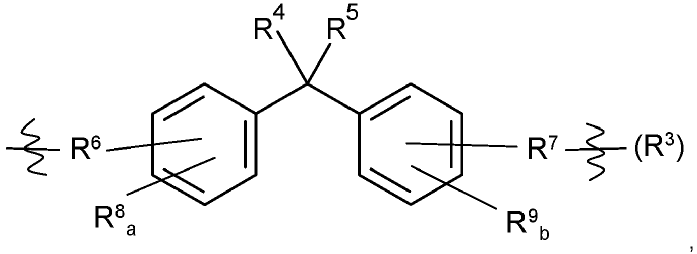 wobei
R⁴ und R⁵ unabhängig voneinander ausgewählt sind aus H, -C₁₋₄-Alkyl, -CF₃, phenyl, oder
R⁴ und R⁵ eine Ringstruktur bilden, umfassend eine Kohlenwasserstoffkette umfassend 4 bis 8 Kohlenstoffatome,
R⁶ ausgewählt ist aus -[(CH₂)ₚ-O-]_{q}-,
R⁷ ausgewählt ist aus -O-[(CH₂)ᵥ-O-]_{w}-,
R⁸ₐ und R⁹_{b} unabhängig voneinander ausgewählt sind aus -C₁₋₄-Alkyl, phenyl,
Y ausgewählt ist aus -O-C(=O)-NR²⁰- und -NR²¹-C(=O)-O-,
R²⁰ ein C₁₋₄-Alkyl oder H ist,
m, p, v zwischen 1 und 4, insbesondere 1 oder 2, ist,
n, q, w zwischen 1 und 4 ist,
r, t, x zwischen 1 und 4 ist, insbesondere 1 oder 2,
s zwischen 1 und 10 ist,
a 0 oder 1 ist,
b 0 oder 1 ist, und
R³ gleich -H, Acrylat oder Methacrylat ist.

8. Das 3D-Druckharz nach einem der vorhergehenden Ansprüche, wobei das Oligomer aus Monomeren gemäß Anspruch 7 gebildet ist.

9. Das 3D-Druckharz nach einem der vorhergehenden Ansprüche, wobei der Initiator ein Photoinitiator ist, insbesondere ein Photoinitiator ausgewählt aus
∘ einer Verbindung der Benzoine und Benzoinether, insbesondere Benzoin, Benzoinmethylether, Benzoinethylether und Benzoinisopropylether, Benzoinphenylether und Benzoinacetat,
∘ einer Verbindung der Acetophenone, insbesodere Acetophenon, 2,2-Dimethoxyacetophenon, und 1,1-Dichloracetophenon,
∘ einer Verbindung der Benzile und Benzilketale, insbesondere Benzil, Benzildimethylketal und Benzildiethylketal,
∘ einer Verbindung der Anthrachinone, insbesondere 2-Methylanthrachinon, 2-Ethylanthrachinon, 2-tert.-Butylanthrachinon, 1-Chloranthrachinon und 2-Amylanthrachinon,
∘ einer Verbindung der Triphenylphosphine und Benzoylphosphinoxide, insbesondere Triphenylphosphine, 2,4,6-Trimethylbenzoyldiphenylphosphinoxid (Luzirin TPO) und Bis(2,4,6-trimethylbenzoylphenyl)-phosphinoxid,
∘ einer Verbindung der Benzophenone, insbesondere Benzophenon und 4, 4'-Bis-(N, N'-dimethylamino)-benzophenon,
∘ einer Verbindung der Thioxanthone und Xanthone, insbesondere Thioxanton und Xanthon,
∘ einem Acridinderivat, Phenazinderivat, Quinoxalinderivat
∘ 1-Phenyl-1,2-propandion-2-O-benzoyloxim,
∘ einer Verbindung der 1-Aminophenylketone oder 1-Hydroxyphenylketone, insbesondere 1-Hydroxycyclohexylphenylketon, Phenyl-(1-hydroxyisopropyl)-keton und 4-Isopropylphenyl-(1-hydroxyisopropyl)-keton.

10. Das 3D-Druckharz nach einem der vorhergehenden Ansprüche, wobei das 3D-Druckharz einen oder mehrere Zusätze ausgewählt aus einem Inhibitor, einem Stabilisator, einem Füllstoff, einem Farbmittel, einem UV-Blocker (Zusatz zur Steuerung der Durchhärtungstiefe) umfasst.

11. Ein Polymer, insbesondere hergestellt unter Verwendung des 3D-Druckharzes nach einem der Ansprüche 1 bis 10, umfassend
- polymerisierte Monomere und/oder Oligomere nach einem der Ansprüche 1 bis 10, und
- eine gesättigte Fettsäure oder einen Carbonsäureester.

12. Ein Grünling, insbesondere hergestellt unter Verwendung des 3D-Druckharzes nach einem der Ansprüche 1 bis 10, umfassend
- polymerisierte Monomere und/oder Oligomere nach einem der Ansprüche 1 bis 10, wobei 60 % bis 80 % der Doppelbindungen der Acrylat- und/oder Methacrylatuntereinheiten umgesetzt worden sind, und
- eine gesättigte Fettsäure oder einen Carbonsäureester.

13. Ein Kit-of-parts-System umfassend
- ein 3D-Druckharz nach einem der Ansprüche 1 bis 10 zur Herstellung eines Polymers mit einem Separationseffekt, und
- eine Pulverkomponente und eine Flüssigkomponente zur Herstellung eines Kunststoffes, wobei
∘ die Pulverkomponente ein Polymerpulver, insbesondere ein Polymerpulver umfassend mindestens eine Acrylat- oder Methacrylat-Untereinheit, umfasst, und
∘ die Flüssigkomponente ein Monomer, insbesondere ein Monomer umfassend mindestens eine Acrylat- oder Methacrylat-Untereinheit, umfasst.

14. Ein Verfahren zur Herstellung eines Formteils mit einem Separationseffekt, insbesondere eines Dentalmodells mit einem Separationseffekt, wobei das 3D-Druckharz nach einem der Ansprüche 1 bis 10 verwendet und ausgehärtet wird, insbesondere unter Verwendung eines 3D-Druckers.

15. Ein Verfahren zur Herstellung eines Formteils, wobei
- in einem ersten Schritt das Verfahren nach Anspruch 14 durchgeführt wird, um ein erstes Formteil zu erhalten, und
- in einem zweiten Schritt das erste Formteil verwendet wird, um ein zweites Formteil zu erhalten, das mindestens abschnittweise komplementär zum ersten Formteil ist, wobei das zweite Formteil aus einem Kunststoff gefertigt wird, insbesondere unter Verwendung einer Pulverkomponente und einer Flüssigkomponente, wobei
∘ die Pulverkomponente ein Polymerpulver, insbesondere ein Polymerpulver umfassend mindestens eine Acrylat- oder Methacrylat-Untereinheit, umfasst, und
∘ die Flüssigkomponente ein Monomer, insbesondere ein Monomer umfassend mindestens eine Acrylat- oder Methacrylat-Untereinheit, umfasst,
oder
- in einem ersten Schritt ein erstes Formteil aus einem Kunststoff gefertigt wird, insbesondere unter Verwendung einer Pulverkomponente und einer Flüssigkomponente, wobei
∘ die Pulverkomponente ein Polymerpulver, insbesondere ein Polymerpulver umfassend mindestens eine Acrylat- oder Methacrylat-Untereinheit, umfasst, und
∘ die Flüssigkomponente ein Monomer, insbesondere ein Monomer umfassend mindestens eine Acrylat- oder Methacrylat-Untereinheit, umfasst,
- in einem zweiten Schritt das erste Formteil verwendet wird, um ein zweites Formteil zu erhalten, das mindestens abschnittweise komplementär zum ersten Formteil ist, wobei das zweite Formteil unter Verwendung des 3D-Druckharzes nach einem der Ansprüche 1 bis 10 hergestellt wird.
